# EUROPÄISCHE PATENTSCHRIFT

(11) **EP 2 922 948 B1**
(45) Veröffentlichungstag und Bekanntmachung des Hinweises auf die Patenterteilung: **21.06.2017**
(21) Anmeldenummer: 12821104.2
(22) Anmeldetag: 26.11.2012
(51) Int. Cl.: C12M 1/107, C12M 3/00, E04H 7/06, F17C 3/00

(54) **VORRICHTUNG ZUR GASDICHTEN ABDECKUNG VON LAGERBEHÄLTERN OHNE INNENSTÜTZE**
DEVICE FOR THE GAS-TIGHT COVERING OF STORAGE CONTAINERS WITHOUT AN INNER SUPPORT
DISPOSITIF DE RECOUVREMENT ÉTANCHE AUX GAZ DE RÉSERVOIRS DE STOCKAGE SANS SUPPORT INTÉRIEUR

(43) Veröffentlichungstag der Anmeldung: 30.09.2015
(73) Patentinhaber: S+B Service und Betrieb GmbH, 86720 Nördlingen (DE)
(72) Erfinder: WERMUTH, Gert, 86720 Nördlingen (DE); AUERBACH, Hans-Joachim, 08066 Zwickau (DE)
(74) Vertreter: Auerbach, Bettina
(86) Internationale Anmeldenummer: PCT/DE2012/001130
(87) Internationale Veröffentlichungsnummer: WO 2014/079401

(56) Entgegenhaltungen:
- EP-A1- 1 801 037
- EP-A1- 2 428 558
- DD-A1- 274 021
- DE-A1- 1 434 598
- DE-A1- 1 684 612
- DE-A1- 1 903 468
- DE-A1-102010 008 897
- DE-U1-202006 014 148
- GB-A- 986 785
- US-A- 3 205 665

## Beschreibung

Die Erfindung betrifft eine Vorrichtung zur gasdichten Abdeckung von Lagerbehältern ohne Innenstütze, wie sie zur Vermeidung von Energieverlusten und/oder von Schadstoffemissionen bei der Lagerung von organischen und/oder anorganischen Fluiden benötigt werden. Solche Vorrichtungen finden insbesondere in Tierzuchtbetrieben bei der Lagerung flüssiger Exkremente oder bei der aeroben und anaeroben Abwasser- und/oder Abfallbehandlung Verwendung, um die Emission von Gerüchen und Luftschadstoffen zu vermeiden sowie zur Erfassung und stofflichen und energetischen Verwertung von energiereichen bzw. von nährstoffhaltigen Gasen.

Um die Abdeckung von Lagerbehältern ohne Verfügbarkeit einer Innenstütze zu realisieren, wurden bereits mehrere technische Lösungen vorgeschlagen.

So offenbart die DD 274021 A1 einen Behälter zur anaeroben Behandlung von Gülle, der durch eine gasdichte Plane abgedeckt ist, wobei diese Plane einerseits mit dem Behälterrand verbunden ist und ihre konfektionierte Form dadurch erhält, dass sie mit einem gewichtsbelasteten Seiltragwerk durch eine Vielzahl von flexiblen Halteelementen verbunden ist. Diese praktisch ch kaum realisierbare technische Idee muss daran scheitern, dass im Falle des Verlassens der Endstellung der vorkonfektionierten Abdeckplane bei Windbelastung unbeherrschbare Segelwirkungen auftreten.

Mit der EP 1801037 A1 wird eine Abdeckung für Güllebehälter, Biogasspeicher und dergleichen bekannt gemacht, die im wesentlichen durch Schwimmkörper mit zwei unterschiedlichen Funktionen gekennzeichnet ist. Ein ringförmig ausgebildeter Schwimmkörper ist als lageveränderliches Abdichtelement zwischen Flüssigkeitsoberfläche und Behälterwand vorgesehen.

Über dieses Abdichtelement ist die konfektionierte Abdeckplane gespannt, wobei die Spannung durch mit der Abdeckplane am Umfang verbundene Ballastelemente erzielt wird, die in die Behälterflüssigkeit eintauchen. Eine weitere Art von Schwimmkörpern hält die Abdeckplane in der konfektionierten geometrischen Figur.
Im Falle der Verminderung des Gasdruckes zwischen der Flüssigkeitsoberfläche und der Abdeckplane kommt es infolge der Wirkung der Ballastelemente zum tieferen Eintauchen der Abdeckplane in die Behälterflüssigkeit. Grundsätzlich kann in speziellen Einsatzfällen auf den Einsatz von schwimmenden Stützkörpern verzichtet werden, insbesondere dann, wenn die Abdeckplane halbkugelförmig oder kugelabschnittsförmig konfektioniert wurde. Allerdings ist diese technische Lösung mit wenigstens drei Mängeln behaftet. So lässt sich die vollständige Abdeckung des Behälters wegen des technisch erforderlichen Abstandes des abdichtenden ringförmigen Schwimmkörpers zur Behälterwand im praktischen Betrieb ohne mögliche Beschädigungen der Abdeckplane nicht erreichen, woraus Gasemissionen und im Falle der Speicherung von Biogas auch Energieverluste resultieren. Im Falle des erwünschten Fernhaltens von Oberflächenwasser ist auch der Eintrag des auf die Abdeckplane auftreffenden Regens in den abgedeckten Behälter als Mangel zu verzeichnen. Angesichts der Tatsache, dass flexible Kunststoffplanen als bevorzugtes Abdeckmaterial für eine Reihe von Gasmolekülen nur mit beachtlichen Diffusionswerten verfügbar sind, lassen sich die diffundierenden Gase technisch praktisch nicht erfassen und bei Erfordernis auch nicht nachbehandeln.
Schließlich stellt die Montage der Ballastelemente ein Problem dar, weil sich die benötigten Konstruktionen aus Stahlbeton oder Naturstein kaum im gefüllten Behälter montieren lassen.

Die WO 2008/031593 A1 kennzeichnet einen Gasspeicher, der im Falle eines erläuterten Ausführungsbeispiels in Verbindung mit einem abzudeckenden Flüssigkeitsbehälter zum Einsatz kommen kann. Die Abdeckfolie ist dabei an einer starren Deckenkonstruktion befestigt, die ihrerseits an außerhalb des abzudeckenden Behälters angeordneten Halteelementen vertikal verschieblich fixiert ist. Die Abdeckfolie ist außerdem mit dem Behälterrand gasdicht verbunden.
Im Falle des Absenkens der Deckenkonstruktion infolge eines abnehmenden Speicherinhaltes oder eines geringeren Gasdruckes faltet sich der zylindrische Teil der Abdeckplane zwischen der Deckenkonstruktion und dem Behälterrand zusammen, ohne die dichte Behälterabdeckung in Frage zu stellen.

Die Nachteile dieser technischen Lösung bestehen neben den Maßnahmen, die gegen die an der gefalteten zylindrischen Behälterabdeckung angreifenden Windkräfte realisiert werden müssen, in den Wirkungen aus der einschaligen Abdeckung mit den möglichen Gasemissionen. Außerdem stellte der Mechanismus für das Anheben und Absenken der Deckenkonstruktion eine kosten- und wartungsintensive Anlagenkomponente dar.

Die Weiterentwicklung eines abgedeckten Flüssigkeitsbehälters mit einem zusätzlichen Gasraum wird mit der DE 102010008897 B4 bekannt gemacht. Eine spezielle Behälterkonstruktion ermöglicht dabei das mit einem Gasfüllvorgang einhgergehende Aufrichten der flexiblen Behälterwand, wobei mit der Behälterwand verbundene Verstärkungsstreben nach vollständiger Aufrichtung in vorbereiteten Positionen des Fundamentes verankert werden. Gleichzeitig werden ausgewählte Punkte des oberen Randes der Behälterwand mit Auflageelementen so miteinander verbunden, dass diese für eine sich absenkende Gasspeicherfolie unter der gegebenenfalls luftgestützten Wetterschutzmembran einen Auflagerost bilden, ohne durch eine Mittelstütze stabiliert zu werden.
Für das Nachrüsten bestehender Behälter ohne Mittelstütze mit einer gasdichten Abdeckung ist auch diese technische Lösung nicht geeignet. Hinzu kommt, dass bei größeren Behälterdurchmessern das Installieren von Elementen eines Auflagerostes ohne den Einsatz zusätzlicher Mittelstützen an konstruktive Grenzen stößt.

Die Aufgabe der Erfindung besteht deshalb in der Entwicklung einer technischen Lösung für die gasdichte Abdeckung von großvolumigen offenen Lagerbehältern, mit deren Hilfe die Mängel des bekannten Standes der Technik überwunden werden. Insbesondere geht es um eine kostengünstige technische Lösung, die mit bekannten Mitteln an bestehenden Lager- oder Prozessbehältern nachgerüstet werden kann, um die Emissionsvermeidung von Gerüchen oder Luftschadstoffen sowie das Vermeiden von Energieverlusten im Fall der Lagerung brennbarer Gase zu erreichen.

Die Aufgabe wird erfindungsgemäß durch die Merkmale des Anspruchs 1 gelöst. Vorteilhafte Ausgestaltungen der Erfindung werden durch die Merkmale der Unteransprüche beschrieben.

Danach wird eine gasdichte Abdeckung von Lagerbehältern ohne Innenstütze, die aus einer einfachen oder zweifachen flexiblen Membrane besteht, erhalten, wenn die eingesetzten Membranen etwa im Bereich der senkrechten Behälterachse gegen horizontale Verschiebungen fixiert sind. Diese grundsätzliche Anordnung fußt auf der Beobachtung der oft einseitigen Auflage einer installierten Gasspeichermembran bei geringem Gasfüllstand auf dem Schutzgitter oberhalb des maximalen Füllstandes der im Lagerbehälter befindlichen Prozessflüssigkeit, das bisher aus starren oder flexiblen Elementen zwischen der Behälterwand und dem Kopf einer Mittelstütze gebildet wird. Im Falle des Einsatzes einer Doppelmembran haben weder die untere Membran zur Aufnahme anfallender Prozessgase noch die obere und regelmäßig luftgestützte Wetterschutzmembran einen Kontakt mit der Mittelstütze.
Insbesondere die so genannte Gasspeichermembran könnte auf diese Weise bei geringeren Gasfüllständen die halbkugel- oder kugelabschnitssförmig konfektionierte Geometrie verlassen und teilweise in die Flüssigkeit des Lagerbehälters eintauchen, wenn die rostförmige Auflagerkonstruktion zwischen Mittelstütze und Behälterwand nicht verfügbar wäre.
Die den annähernden Mittelpunkt der Behälterabdeckung fixierende Haltekonstruktion ist oberhalb der Abdeckmembranen angeordnet, wobei die Lage der mittlere Verankerung der Abdeckmembranen wenigstens zu zwei Verankerungspunkten gesichert ist.
Die Stützelemente für die Haltekonstruktionen sind außerhalb des abzudeckenden Lagerbehälters angeordnet, womit für bestehende Lagerbehälter im Falle der vorgesehenen nachträglichen Abdeckung das Entleeren, die Verstärkung des Behälterbodens und das Errichten einer Mittelstütze mit der Auflagerkonstruktion zur Vermeidung von Kontakten zwischen der Gasspeichermembran und der im Lagerbehälter befindlichen Prozessflüssigkeit entfallen kann.
Im Falle des Einsatzes einer zweischaligen Abdeckmembran werden die Membranen außer an der Behälterwand des abzudeckenden Lagerbehälters auch im Bereich der Behälterachse miteinander verbunden.
Das Verbindungselement für die beiden Abdeckmembranen im Bereich der Behälterachse ist ein flexibles Zugelement.
Außerdem ist im Bereich zwischen dem zu lagernden Medium und der unteren Abdeckmembran etwa in der Behältermitte ein Ballastelement angeordnet.

Zur Vermeidung von Gasverlusten und der Emissionen von Gerüchen und Luftschadstoffen sind die eingesetzten Abdeckmembranen im oberen Bereich der Behälterwand mit dieser gasdicht verbunden.

In einer bevorzugten Ausführungsform sind die Stützelemente für die Haltekonstruktionen mit der Behälterwand des abzudeckenden Lagerbehälters verbunden.

Für die Haltekonstruktionen werden bevorzugt starre Elemente, beispielsweise Vollwand- oder Fachwerkträgerkonstruktionen, und/oder flexible Elemente , beispielsweise Seil- oder Gurtkonstruktionen, aus Metall oder verstärkten Kunststoffen eingesetzt.

Insbesondere die einzusetzenden Stützelemente und/oder die Haltekonstruktionen können mit Blitzableiterfunktionen ausgestattet sein.

Auch das vorgeschlagene flexible Zugelement ist eine Seil- oder Gurtkonstruktion aus faserverstärktem Kunststoff.

Das unterhalb der unteren Membran, die in erster Linie die Funktion der Gasspeichermembran besitzt, angeordnete Ballastelement besteht bevorzugt aus Stahlbeton, Stahl oder aus einem mit Naturstein gefülltem Behälter aus Stahl oder verstärktem Kunststoff.

In einer weiteren Azusführungsvariante ist vorgesehen, im Bereich des oberen Randes des abzudeckenden Lagerbehälters ein mit dem oberen Rand des Lagerbehälters verbundene Auflagekonstruktion einzusetzen, die aus wenigstens drei in der Behälterachse zusammengeführten Auflageelementen besteht, wobei zwischen den benachbarten Auflageelementen starre oder flexible Verbindungselemente angeordnet sind, die für wenigstens eine der eingesetzten Abdeckmembranen vorgesehen sind.

Weiterhin können an der Auflagekonstruktion Einrichtungen für die Behälterfüllung und/oder für die Gasentnahme angeordnet sein.

Im Falle des Einsatzes einer zweischaligen Abdeckmembran ist die äußere Abdeckmembran als luftgestützte Konstruktion ausgebildet.

Dagegen ist im Falle des Einsatzes einer einschaligen Abdeckmembran die Abdeckmembran als prozessgasgestützte Konstruktion ausgebildet.

Die Vorteile der erfindungsgemäßen technischen Lösung bestehen in erster Linie in der nun verfügbaren Möglichkeit, der Forderung nach vollständiger Abdeckung bisher offen betriebener Prozessbehälter in der Abwassertechnik, in der Chemieindustrie und auch in Anlagen zur Nassvergärung biogener Einsatzstoffe auch dann nachkommen zu können, wenn das Nachrüsten solcher Behälter mit Mittelstützen aus technischen, logistischen und/oder wirtschaftlichen Gründen auszuschließen ist. Je nach Baugrundbeschaffenheit und Platzverhältnissen werden sich die Stütz- und Haltekonstruktionen außerhalb der abzudeckenden Behälter und oberhalb der einzusetzenden Abdeckungen befinden und unterschiedlich ausgebildet sein. Immer geht es jedoch darum, den etwaigen Mittelpunkt der äußeren Abdeckmembran in erfindungsgemäßer Weise höhen- und lagemäßig zu fixieren und insbesondere im Falle des Einsatzes einer darunter angeordneten zweiten Membrane, beispielswiese zum Zwecke der Gasspeicherung, durch ein flexibles Element mit dem etwaigen Mittelpunkt dieser Membran und einem unter dieser Membran angeordneten Ballastelement zu verbinden. Der auf diese Weise in horizontaler Richtung fixierte Mittelpunkt der Abdeckmembranen vermeidet die einseitige Verschiebung einer nicht vollständig gefüllten Membran, was die Verfügbarkeit eines Auflagerostes zur Vermeidung des Eintauchens der Abdeckung in die gelagerte Flüssigkeit überflüssig macht.
Schließlich besteht im Falle einer zweischaligen Abdeckung die Möglichkeit, die über ein Überdruckventil aus der luftgestützten Witterungsschutzmembran austretende Stützluft so nachzubehandeln, dass sie von luftfremdem Stoffen entfrachtet werden kann.

Die Erfindung soll nachstehend mit Ausführungsbeispielen näher erläutert werden.
In der beigefügten Zeichnung zeigen
- Fig. 1:: die schematische Darstellung der in der Behälterachse angeordneten Vorrichtungskomponenten einer zweischaligen Behälterabdeckung;
- Fig. 2:: die schematische Schnittdarstellung einer zweischaligen Behälterabdeckung bei mittlerer Füllung der Gasspeichermembran durch Prozessgas mit der Fixierung des Mittelpunktes durch ein Seiltragwerk;
- Fig. 3:: die schematische Draufsicht auf eine Behälterabdeckung mit der Fixierung des Mittelpunktes durch ein dreigliedriges Seiltragwerk;
- Fig. 4:: die schematische Schnittdarstellung einer zweischaligen Behälterabdeckung bei maximaler Füllung der Gasspeichermembran durch Prozessgas mit der Fixierung des Mittelpunktes durch eine starre Bogenkonstruktion;
- Fig. 5:: die schematische Draufsicht auf eine Behälterabdeckung mit der Fixierung des Mittelpuktes durch eine zweigliedrige Bogenkonstruktion;
- Fig. 6:: die schematische Schnittdarstellung einer zweischaligen Behälterabdeckung einer zweischaligen Behälterabdeckung bei minimaler Füllung der Gasspeichermembran durch Prozessgas mit der Fixierung des Mittelpunktes durch eine starre Brückenkonstruktion;
- Fig. 7:: die schematische Draufsicht auf eine Behälterabdeckung mit der Fixierung des Mittelpuktes durch eine zweigliedrige Brückenkonstruktion;
- Fig. 8:: die schematische Schnittdarstellung einer einschaligen prozessgasgestützten Abdeckmembran mit Einrichtungen für die Behälterfülllung und für die Gasentnahme;
- Fig. 9:: die schematische Schnittdarstellung einer zweischaligen Abdeckmembran mit einer Auflagekonstruktion für die Gasspeichermembran;
- Fig. 10:: die schematische Draufsicht auf eine Auflagekonstruktion für die Gaspeichermembran.

### Ausführungsbeispiel 1:

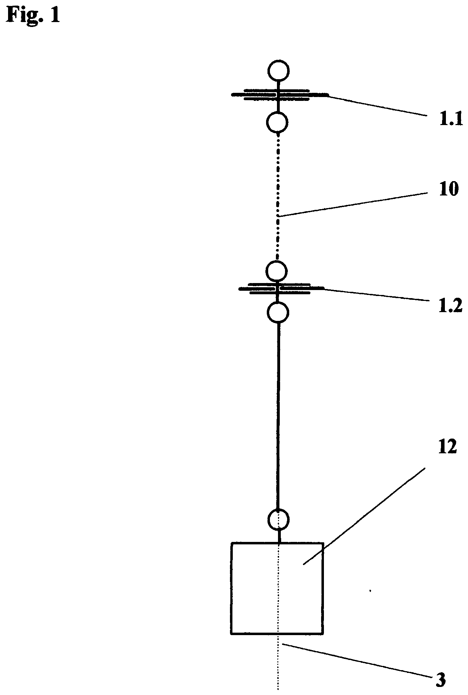

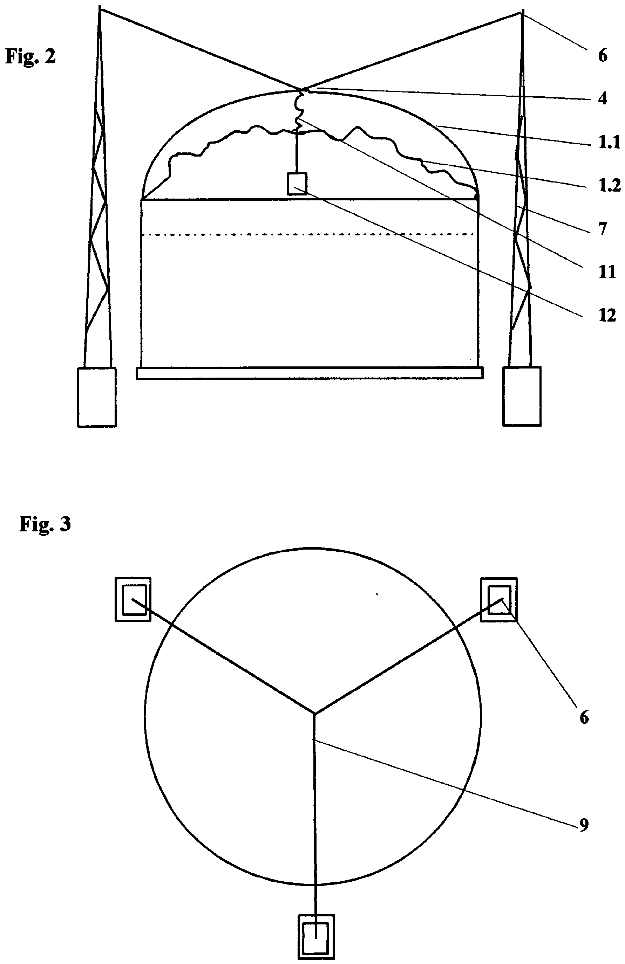

Gemäß der Figuren 1 bis 3 besteht eine zweischalige Behälterabdeckung aus zwei flexiblen Abdeckmembranen 1.1, 1.2 aus faserverstärkten Kunststoffen, die kugelabschnittsförmig konfektioniert wurden. Etwa in der senkrechten Behälterachse 3 sind beide Abdeckmembranen 1.1, 1.2 zwischen jeweils zwei so genannte Klöpperböden aus Edelstahl eingespannt. An den Klöpperböden sind stabile Befestigungsösen positioniert. Die oberhalb der äußeren Abdeckmembran 1.1 angeordnete Befestigungsöse bildet die Verankerung 4 für die Fixierung der äußeren Behälterabdeckung 1.1 an der Haltekonstruktion 5. Zwischen den Befestigungsösen unterhalb der äußeren Abdeckmembran 1.1 und oberhalb der inneren Abdeckmambran 1.2 ist ein flexibles Zugelement 11 angeordnet, damit sich die innere Abdeckmembran 1.2 bei maximaler Füllung mit Prozessgas weitgehend der äußeren Abdeckmembran 1.1 annähern kann. In diesem Fall ist die Mittelpunktlage der inneren Abdeckmembran 1.2 durch die Konfektionierung dieses Abdeckelementes gewährleistet. An der Befestigungsöse unterhalb der inneren Abdeckmembran 1.2 ist das Verbindungselement 10 angeordnet, das das Ballastelement 12 trägt. Bei teilweiser oder bei minimaler Füllung der innereren Abdeckmembran 1.2 mit Prozessgas könnte sich der Mittelpunkt der inneren Abdeckmembran 1.2 von der senkrechten Behälterachse 3 entfernen, wenn sich unter Wirkung des Ballastelementes 12 das flexible Zugelement 11 zwischen der äußeren Abdeckmembran 1.1 und der inneren Abdeckmembran 1.2 nicht straffen würde. Damit werden in den kritischen Betriebssituationen mit schlaffer Beschaffenheit der inneren Abdeckmembran 1.2 die Mittelpunkte beider Abdeckmembranen 1.1, 1.2 unterhalb der Verankerung 4 an der Haltekonstruktion 5 etwa in der Nähe der senkrechten Behälterachse 3 gehalten. Die Haltekonstruktion 5 besteht aus einem dreigliedrigen Seiltragwerk. Die flexiblen Elemente 9 dieses Tragwerkes stehen mit den Verankerungspunkten 6 von drei Stützelementen 7 in Verbindung. Diese Stützelemente 7 sind als außerhalb des abgedeckten Behälters angeordnete Pylonen ausgebildet. Diese Gestaltung der Beälterabdeckung erlaubt es, bei vollständigem Erhalt der technischen, energetischen und emissionseitigen Vorteile einer Behälterabdeckung mittels Doppelmembran auf eine Mittelstütze für ein sicherndes Balken- oder Gurttragwerk für die mitunter schlaffe innere Abdeckmembrane 1.2 zu verzichten. Dabei kann es ohne technische Konsequenzen wohl zum Kontakt oder zum Eintauchen des Ballastelementes 12 in das im Behälter gelagerte flüssige Medium kommen, nicht jedoch zum Kontakt der inneren Abdeckmembran 1.2 mit dieser Flüssigkeit.

### Ausführungsbeispiel 2:

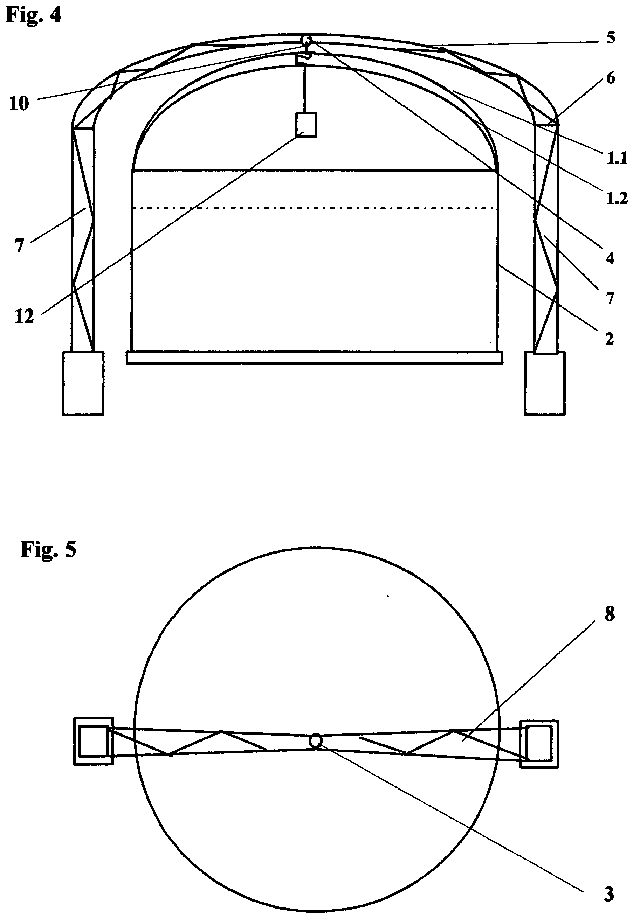

Gemäß der Figuren 4 und 5 ist eine zweischalige Behälterabdeckung, die aus einer äußeren 1.1 und einer inneren Abdeckmembran 1.2 besteht, grundsätzlich wie im Beispiel 1 gestaltet. Die Verankerung 4 der beiden Abdeckmembranen 1.1, 1.2 erfolgt hier jedoch an einer Haltekonstruktion 5, die aus zwei starren Elementen 8 in Form einer bogenförmigen Trägerkonstruktion besteht. Diese bogenförmige Trägerkonstruktion ist an zwei Verankerungspunkten 6 mit zwei Stützelementen 7 verbunden, wobei diese Stützelemete 7 außerhalb des abzudeckenden Behälters angeordnet sind.

### Ausführungsbeispiel 3:

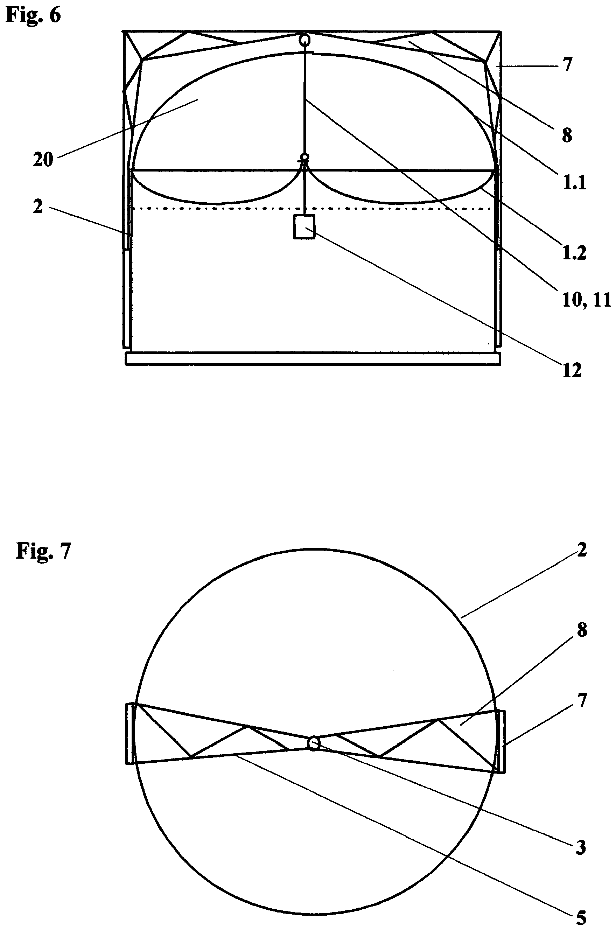

Gemäß der Figuren 6 und 7 ist eine zweischalige Behälterabdeckung wie im Beispiel 2 gestaltet. Während es sich bei der äußeren Abdeckmembran 1.1 um eine luftgestützte Konstruktion 20 handelt, ist die innere Abdeckmembran 1.2 eine prozessgasgestützte Konstruktion 21. Die beiden Abdeckmembranen 1.1, 1.2 sind etwa in der senkrechten Behälterachse 3 an der Verankerung 4 einer Haltekonstruktion 5 befestigt, womit insbesondere die Mittelpunktlage beider Abdeckmembranen 1.1, 1.2 auch dann etwa im Bereich der senkrechten Behälterachse 3 gehalten wird, wenn die innere Abdeckmembran 1.2 bei geringer Füllung mit Prozessgas eine schlaffe Beschaffenheit aufweist und bei einseitiger Verschiebung aus der Mittelpunktlage anderenfalls zumindest teilweise Kontakt mit der im Behälter gelagerten Flüssigkeit bekommen könnte. Bei der Haltekonstruktion 5 handelt es sich um eine Brückenkonstruktion aus zwei starren Elementen 8, die an den Verankerungspunkten 6 von zwei Stützelementen 7 befestigt sind, wobei diese Stützelemente außerhalb des Behälters angeordnet sind.

### Ausführungsbeispiel 4:

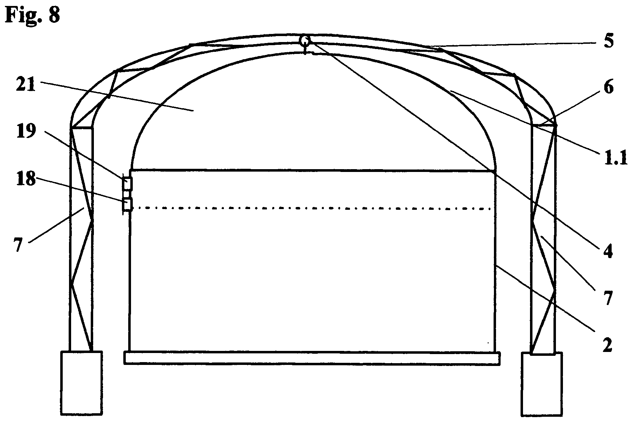

Gemäß der Figur 8 besteht eine Behälterabdeckung ohne Mittelstütze aus einer einschaligen Abdeckmembran 1.1, die als prozessgasgestützte Konstruktion 21 ausgebildet ist. Die Mittelpunktlage der Abdeckmembran 1.1 wird dadurch gesichert, dass die Abdeckmembran 1.1 an der Verankerung 4 einer Haltekonstruktion 5 befestigt ist. Die Haltekonstruktion 5 ist wie im Beispiel 3 gestaltet. Auch bei nachlassendem Druck des die Abdeckmembran 1.1 stützenden Prozessgases kommt es wegen der Befestigung des Mittelpunktes der Abdeckmembran 1.1 an der Verankerung 4 der Haltekonstruktion 5 nicht zum Kontakt der Abdeckmembran 1.1 mit der Flüssigkeit im Lagerbehälter. Wie bei allen beschriebenen Beispielen sind im oberen Bereich der Behälterwand 2, vorzugsweise oberhalb des maximalem Füllstandes der zu lagernden Prozessflüssigkeit, sowohl die Einrichtung für die Behälterfüllung 18 als auch die Einrichtung für die Gasentnahme 19 angeordnet.

### Ausführungsbeispiel 5:

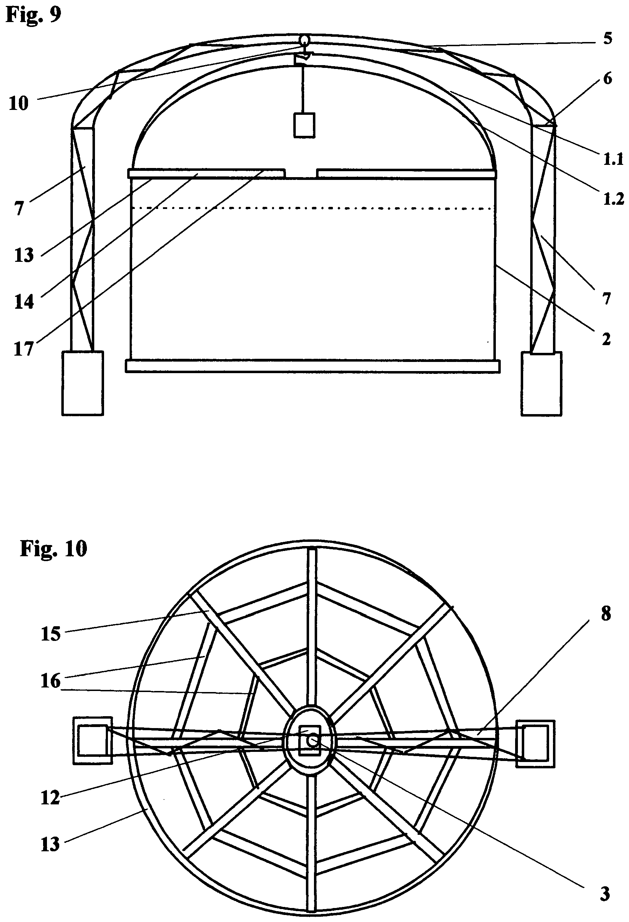

Gemäß der Figuren 9 und 10 ist eine Behälterabdeckung ohne Mittelstütze analog des Beispiels 2 mit einer zweischaligen Abdeckmembran 1.1, 1.2 azusgeführt. Wegen der ausschließbaren Einseitigkeit der Verlagerung der inneren Abdeckmambran 1.2 sind hohe mechanische Belastungen einer Auflagekonstruktion 14 nicht zu befürchten. Deshalb kann in vielen Fällen eine derartige Vorsorgemaßnahme durchaus ohne belastbare Mittelstütze eingesetzt werden. Dazu ist auf dem oberen Behälterrand 13 eine Auflagekonstruktion aus Leichtbauelementen in Form von Holzbalken, Leichtmetallprofilen oder faserverstärkten Kunststoffprofilen befestigt, die aus radial angeordneten Auflageelementen 15 und zwischen diesen Auflageelementen angeordneten Verbindungselementen 16 besteht. Bei Verfügbarkeit dieser Auflagekonstruktion 14 ragt die in bestimmten Betriebssituationen schlaffe innere Abdeckmembran 1.2 nicht in den von der Behälterflüssigkeit nicht berührten Teil des abgedeckten Lagerbehälters hinein, sondern legt sich auf die Oberkante 17 der Auflageelemente 15 und gegebenenfalls auch auf die Oberkanten der Verbindungselemente 16 auf.

### Bezugszeichenliste

- 1.1: - äußere Abdeckmembran
- 1.2: - innere Abdeckmembran
- 2: - Behälterwand
- 3: - Behälterachse
- 4: - Verankerung
- 5: - Haltekonstruktion
- 6: - Verankerungspunkt
- 7: - Stützelement
- 8: - starre Elemente
- 9: - flexible Elemente
- 10: - Verbindungselement
- 11: - flexibles Zugelement
- 12: - Ballastelement
- 13: - oberer Behälterrand
- 14: - Auflagekonstruktion
- 15: - Auflageelement
- 16: - Verbindungselement
- 17: - Oberkante des Auflageelementes
- 18: - Einrichtung für die Behälterfüllung
- 19: - Einrichtung für die Gasentnahme
- 20: - luftgestützte Konstruktion
- 21: - prozessgasgestützte Konstruktion

## Patentansprüche

1. Vorrichtung zur gasdichten Abdeckung von Lagerbehältern ohne Innenstütze, bestehend aus mindestens einer einfachen flexiblen Membrane, **gekennzeichnet dadurch**
**dass** die eingesetzten Membranen (1.1, 1.2) im Bereich der Behälterachse (3) gegen horizontale Verschiebungen fixiert sind; **dass** die den mittleren Bereich der Abdeckmembranen (1.1, 1.2) fixierenden Haltekonstruktionen (5) oberhalb der Abdeckmembranen (1.1, 1.2) angeordnet sind;
**dass** die Lage der mittlere Verankerung (4) der Abdeckmembranen (1.1, 1.2) wenigstens zu zwei Verankerungspunkten (6) gesichert ist; **dass** die Stützelemente (7) für die Haltekonstruktionen (5) außerhalb des abzudeckenden Lagerbehälters angeordnet sind;
**dass** das Verbindungselement (10) für mehrere Abdeckmembranen (1.1, 1.2) im Bereich der Behälterachse (3) ein flexibles Zugelement (11) ist;
**dass** im Bereich zwischen dem zu lagernden Medium und der unteren Abdeckmembran (1.2) ein Ballastelement (12) angeordnet ist.

2. Vorrichtung nach dem Anspruch 1, **gekennzeichnet dadurch, dass** die eingesetzten Abdeckmembranen (1.1, 1.2) im oberen Bereich der Behälterwand (2) mit dieser gasdicht verbunden sind.

3. Vorrichtung nach einem der Ansprüche 1 und 2, **gekennzeichnet dadurch, dass** die Stützelemente (7) für die Haltekonstruktionen (5) mit der Behälterwand (2) des abzudeckenden Lagerbehälters verbunden sind.

4. Vorrichtung nach einem der Ansprüche 1 bis 3, **gekennzeichnet dadurch, dass** die Haltekonstruktionen (5) starre Elemente (8) und/oder flexible Elemente (9) aus Metall oder verstärkten Kunststoffen sind.

5. Vorrichtung nach einem der Ansprüche 1 bis 3, **gekennzeichnet dadurch, dass** die flexiblen Elemente (9) der Haltekonstruktionen (5) metallische oder nichtmetallische Seile oder Gurtkonstruktionen sind.

6. Vorrichtung nach einem der Ansprüche 1 bis 5, **gekennzeichnet dadurch, dass** die Stützelemente (7) und/oder die Haltekonstruktionen (5) mit Blitzableiterfunktionen ausgestattet sind.

7. Vorrichtung nach einem der Ansprüche 1 bis 6, **gekennzeichnet dadurch, dass** das flexible Zugelement (11) eine Seil- oder Gurtkonstruktion aus faserverstärktem Kunststoff ist.

8. Vorrichtung nach einem der Ansprüche 1 bis 7, **gekennzeichnet dadurch, dass** das Ballastelement (12) aus Stahlbeton, Stahl oder aus einem mit Naturstein gefülltem Behälter aus Stahl oder verstärktem Kunststoff besteht.

9. Vorrichtung nach einem der Ansprüche 1 bis 8, **gekennzeichnet dadurch, dass** im Bereich des oberen Randes (13) des abzudeckenden Lagerbehälters ein mit dem oberen Rand (13) des Lagerbehälters verbundene Auflagekonstruktion (14), die aus wenigstens drei in der Behälterachse (3) zusammengeführten Auflageelementen (15) besteht, wobei zwischen den benachbarten Auflageelementen (15) starre oder flexible Verbindungselemente (16) angeordnet sind, für wenigstens eine der eingesetzten Abdeckmembranen (1.1, 1.2) angeordnet ist.

10. Vorrichtung nach einem der Ansprüche 1 bis 9, **gekennzeichnet dadurch, dass** an der Auflagekonstruktion (14) Einrichtungen für die Behälterfüllung (18) und/oder für die Gasentnahme (19) angeordnet sind.

11. Vorrichtung nach einem der Ansprüche 1 bis 10, **gekennzeichnet dadurch, dass** im Falle des Einsatzes einer zweischaligen Abdeckmembran (1.1, 1.2) die äußere Abdeckmembran (1.1) als luftgestützte Konstruktion (20) ausgebildet ist.

12. Vorrichtung nach einem der Ansprüche 1 bis 10, **gekennzeichnet dadurch, dass** im Falle des Einsatzes einer zweischaligen Abdeckmembran (1.1, 1.2) die Membranen außer an der Behälterwand (2) des abzudeckenden Lagerbehälters auch im Bereich der Behälterachse (3) miteinander verbunden sind.

13. Vorrichtung nach einem der Ansprüche 1 bis 11, **gekennzeichnet dadurch, dass** im Falle des Einsatzes einer einschaligen Abdeckmembran (1) die Abdeckmembran (1.2) als prozessgasgestützte Konstruktion (21) ausgebildet bist.

## Claims

1. Apparatus for the gas-tight covering of storage containers without an inner support, comprising a single or double flexible membrane, **characterized in that**
the membranes (1.1, 1.2) used are fixed approximately in the region of the container axis (3) against horizontal displacement;
**in that** the retaining structures (5), which fix the central region of the covering membranes (1.1, 1.2), are arranged above the covering membranes (1.1, 1.2);
**in that** the position of the central anchoring means (4) of the covering membranes (1.1, 1.2) is secured at least at two anchoring points (6);
**in that** the supporting elements (7) of the retaining structures (5) are arranged outside the storage container which is to be covered;
**in that**, in the case of a double-skin covering membrane (1.1, 1.2) being used, the membranes are connected to one another not just on the wall (2) of the storage container which is to be covered but also in the region of the container axis (3);
**in that** the connecting element (10) for the two covering membranes (1.1, 1.2) in the region of the container axis (3) is a flexible tension element (11); and
**in that** a ballast element (12) is arranged in the region between the medium which is to be stored and the lower covering membrane (1.2).

2. Apparatus according to Claim 1, **characterized in that** the covering membranes (1.1, 1.2) used are connected to the container wall (2) in a gas-tight manner in the upper region of said container wall.

3. Apparatus according to either of Claims 1 and 2, **characterized in that** the supporting elements (7) for the retaining structures (5) are connected to the wall (2) of the storage container which is to be covered.

4. Apparatus according to one of Claims 1 to 3, **characterized in that** the retaining structures (5) are rigid elements (8) and/or flexible elements (9) made of metal or reinforced plastics materials.

5. Apparatus according to one of Claims 1 to 3, **characterized in that** the flexible elements (9) of the retaining structures (5) are metallic or neon-metallic cables or belt structures.

6. Apparatus according to one of Claims 1 to 5, **characterized in that** the supporting elements (7) and/ar the retaining structures (5) are provided with lightning-conductor functions.

7. Apparatus according to one of Claims 1 to 6, **characterized in that** the flexible tension element (11) is a cable structure or belt structure made of fibre-reinforced plastics material.

8. Apparatus according to one of Claims 1 to 7, **characterized in that** the ballast element (12) consists of reinforced concrete or steel or comprises a container which is made of steel or reinforced plastics maternal and is filled with natural stone.

9. Apparatus according to one of Claims 1 to 8, **characterized in that** the region of the upper periphery (13) of the storage container which is to be covered has arranged in it a bearing structure (14) which is connected to the upper periphery (13) of the storage container, comprises at least three bearing elements (15) meeting at the container axis (3), rigid or flexible connecting elements (16) being arranged between the adjacent bearing elements (15), and is intended for at least one of the covering membranes (1.1, 1.2) used.

10. Apparatus according to one of Claims 1 to 9, **characterized in that** devices for container filling (18) and/or for gas removal (19) are arranged on the bearing structure (14).

11. Apparatus according to one of Claims 1 to 8, **characterized in that** the single covering membrane or, in the case of a so-called double membrane being used, the outer covering membrane (1.2) is fastened in the manner of a tent in the centre of the container, both on the anchoring means (4) and on the upper container periphery (13), and is in the form of a cone, truncated cone, pyramid or truncated pyramid.

12. Apparatus according to one of Claims 1 to 11, **characterized in that**, in the case of a double-skin covering membrane (1.1, 1.2) being used, the outer covering membrane (1.1) is designed in the form of an air-supported structure (20).

13. Apparatus according to one of Claims 1 to 12, **characterized in that**, in the case of a single-skin covering membrane (1) being used, the covering membrane (1.2) is designed in the form of a process-gas-supported structure (21).

## Revendications

1. Dispositif de recouvrement étanche aux gaz de réservoirs de stockage sans support intérieur, se composant d'une membrane souple simple ou double, **caractérisé en ce que**
les membranes utilisées (1.1, 1.2) sont fixées contre un déplacement horizontal environ dans la région de l'axe du réservoir (3);
les structures de maintien (5) fixant la région centrale des membranes de recouvrement (1.1, 1.2) sont disposées au-dessus des membranes de recouvrement (1.1, 1.2);
1a position de l'ancrage central (4) des membranes de recouvrement (1.1, 1.2) est assurée au moins en deux points d'ancrage (6);
les éléments de support (7) pour les structures de maintien (5) sont disposés à l'extérieur du réservoir de stockage à recouvrir;
en cas d'utilisation d'une membrane de recouvrement à double coque (1.1, 1.2), les membranes sont attachées extérieurement à la paroi de réservoir (2) du réservoir à recouvrir et également l'une à l'autre dans la région de l'axe du réservoir (3);
l'élément de liaison (10) pour les deux membranes de recouvrement (1.1, 1.2) dans la région de l'axe du réservoir (3) est un élément de traction souple (11), et
un élément de ballast (12) est disposé dans la région située entre le fluide à stocker et la membrane de recouvrement inférieure (1.2).

2. Dispositif selon la revendication 1, **caractérisé en ce que** les membranes de recouvrement utilisées (1.1, 1.2) sont attachées de façon étanche aux gaz à la paroi de réservoir (2) dans la région supérieure de celle-ci.

3. Dispositif selon une des revendications 1 et 2, **caractérisé en ce que** les éléments de support (7) pour les structures de maintien (5) sont attachés à la paroi de réservoir (2) du réservoir de stockage à recouvrir.

4. Dispositif selon l'une quelconque des revendications 1 à 3, **caractérisé en ce que** les structures de maintien (5) sont des éléments rigides (8) et/ou des éléments souples (9) en métal ou en matières plastiques renforcées.

5. Dispositif selon l'une quelconque des revendications 1 à 3, **caractérisé en ce que** les éléments souples (9) des structures de maintien (5) sont des structures de câbles ou de sangles métalliques ou non métalliques.

6. Dispositif selon l'une quelconque des revendications 1 à 5, **caractérisé en ce que** les éléments de support (7) et/ou les structures de maintien (5) sont doté(e)s de fonctions de paratonnerre.

7. Dispositif selon l'une quelconque des revendications 1 à 6, **caractérisé en ce que** l'élément de traction souple (11) est une structure de câble ou de sangle en matière plastique renforcée par des fibres.

8. Dispositif selon l'une quelconque des revendications 1 à 7, **caractérisé en ce que** l'élément de ballast (12) se compose de béton armé, d'acier ou d'un récipient en acier ou en matière plastique renforcée rempli de pierre naturelle.

9. Dispositif selon l'une quelconque des revendications 1 à 8, **caractérisé en ce qu'**une structure d'appui (14) attachée au bord supérieur (13) du réservoir de stockage, qui se compose d'au moins trois éléments d'appui (15) réunis sur l'axe du réservoir (3), des éléments de liaison rigides ou souples (16) étant disposés entre les éléments d'appui voisins (15), est disposée dans la région du bord supérieur (13) du réservoir à recouvrir pour au moins une des membranes de recouvrement utilisées (1.1, 1.2).

10. Dispositif selon l'une quelconque des revendications 1 à 9, **caractérisé en ce que** des dispositifs pour le remplissage du réservoir (18) et/ou pour le prélèvement de gaz (19) sont disposés sur la structure d'appui (14).

11. Dispositif selon l'une quelconque des revendications 1 à 8, **caractérisé en ce que** la membrane simple ou, en cas d'utilisation d'une membrane double, la membrane de recouvrement extérieure (1.2) est fixée en forme de tente au centre du réservoir aussi bien à l'ancrage (4) qu'au bord supérieur du réservoir (13) et présente une configuration en forme de tronc de cône, de pyramide ou de tronc de pyramide.

12. Dispositif selon l'une quelconque des revendications 1 à 11, **caractérisé en ce qu'**en cas d'utilisation d'une membrane de recouvrement à double coque (1.1, 1.2), la membrane de recouvrement extérieure (1.1) est réalisée sous la forme d'une structure supportée par l'air (20).

13. Dispositif selon l'une quelconque des revendications 1 à 12, **caractérisé en ce qu'**en cas d'utilisation d'une membrane de recouvrement à coque unique (1), la membrane de recouvrement (1.2) est réalisée sous la forme d'une structure supportée par le gaz de traitement (21).
